(19) Europäisches Patentamt
European Patent Office
Office européen des brevets

(11) Veröffentlichungsnummer : **0 138 176**
**B1**

(12) **EUROPÄISCHE PATENTSCHRIFT**

(45) Veröffentlichungstag der Patentschrift : **04.05.88**

(51) Int. Cl.⁴ : **C 07 D215/04**, C 07 D215/18

(21) Anmeldenummer : **84112009.0**

(22) Anmeldetag : **06.10.84**

(54) **Verfahren zur Herstellung von Chinolinen.**

(30) Priorität : **15.10.83 DE 3337569**

(43) Veröffentlichungstag der Anmeldung : **24.04.85 Patentblatt 85/17**

(45) Bekanntmachung des Hinweises auf die Patenterteilung : **04.05.88 Patentblatt 88/18**

(84) Benannte Vertragsstaaten : **BE CH DE FR GB LI NL**

(56) Entgegenhaltungen : **CH-A- 459 204**

(73) Patentinhaber : **BASF Aktiengesellschaft
Carl-Bosch-Strasse 38
D-6700 Ludwigshafen (DE)**

(72) Erfinder : **Dockner, Toni, Dr.
Grossgasse 6
D-6701 Meckenheim (DE)**
Erfinder : **Hagen, Helmut, Dr.
Max-Slevogt-Strasse 17e
D-6710 Frankenthal (DE)**
Erfinder : **Krug, Herbert
Mussbacher Strasse 49
D-6700 Ludwigshafen (DE)**

**EP 0 138 176 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft ein Verfahren zur Herstellung von Chinolinen durch Umsetzung von aromatischen Aminen mit gegebenenfalls substituierten Acroleinen bei erhöhten Temperaturen in einem Mineralöl.

Chinoline werden nach Skraup aus gegebenenfalls substituiertem Anilin und Glycerin hergestellt (Organikum, S. 561, R.H.E. Manske u. M. Kulka, Org. Reactions 7, 59-98 (1953)).

Das Verfahren hat eine Reihe von Nachteilen und entspricht nicht mehr den heutigen Energie- und Umweltschutzvorstellungen.

1. Pro Mol Anilin sind 3 Mol Glycerin erforderlich.
2. Für einen Teil Anilin müssen 2 bis 3 Teile konz. Schwefelsäure eingesetzt werden, die nach der Reaktion mit Lauge neutralisiert wird. Die zu entsorgende Salzlösung enthält noch sehr viel organischen Kohlenstoff.
3. Die Gewinnung der Chinoline erfolgt durch energieaufwendige Wasserdampfdestillation, an die sich eine Lösungsmittelextraktion anschließt. Das wäßrige Raffinat enthält Lösungsmittel und Produktreste, die entsorgt werden müssen.
4. Die Oxidation des intermediär gebildeten Dihydrochinolins wird mit Nitrobenzol, Arsenpentoxid oder Eisen(III)chlorid vorgenommen.

Die Synthese-Variante nach Doebner-Miller verwendet anstelle von Glycerin Acrolein, ist aber mit den übrigen Nachteilen der Skraup-Synthese belastet.

Gemäß CH-A-459 204 wurde auch schon vorgeschlagen, Chinoline durch Umsetzung von Anilinen mit α, β-ungesättigten Carbonylverbindungen in einem im wesentlichen aus Arsensäure bestehenden Reaktionsmedium herzustellen. Dieses Verfahren hat zumindest den Nachteil der Erfordernis größerer Mengen der teuren und toxikologisch nicht unbedenklichen Arsensäure, so daß eine großtechnische Durchführung sehr problematisch ist.

Es bestand daher die Aufgabe ein Verfahren für die Herstellung von Chinolinen vorzuschlagen, das die genannten Nachteile nicht aufweist und das es gestattet, die Nebenprodukte in einfacher Weise abzutrennen.

Diese Aufgabe wurde erfindungsgemäß dadurch gelöst, daß man ein gegebenenfalls substituiertes Anilin mit Acrolein oder anderen α, β-ungesättigten Aldehyden bei höherer Temperatur in einem hochsiedenden Mineralöl umsetzt. Dabei wird Säure nur in katalytischen Mengen benötigt.

Unter hochsiedenden Mineralölen werden hochsiedende Kohlenwasserstoff-Fraktionen verstanden, d.s. in der Regel Raffinerieprodukte mit einem Siedepunkt über 150 °C, wie Gasöl, Vakuumgasöl, Heizöl S, technisches Weißöl, geschmolzenes Paraffinwachs oder aromatisches Kohlenwasserstofföl. Vorteilhaft wird Vakuumgasöl mit einem Siedepunkt von über 200 °C, insbesondere mit einem Siedebereich zwischen 350 und 500 °C verwendet.

Für die Umsetzung von p-Toluidin mit Methacrolein zu 3,6-Dimethylchinolin lautet die Reaktionsgleichung folgendermaßen :

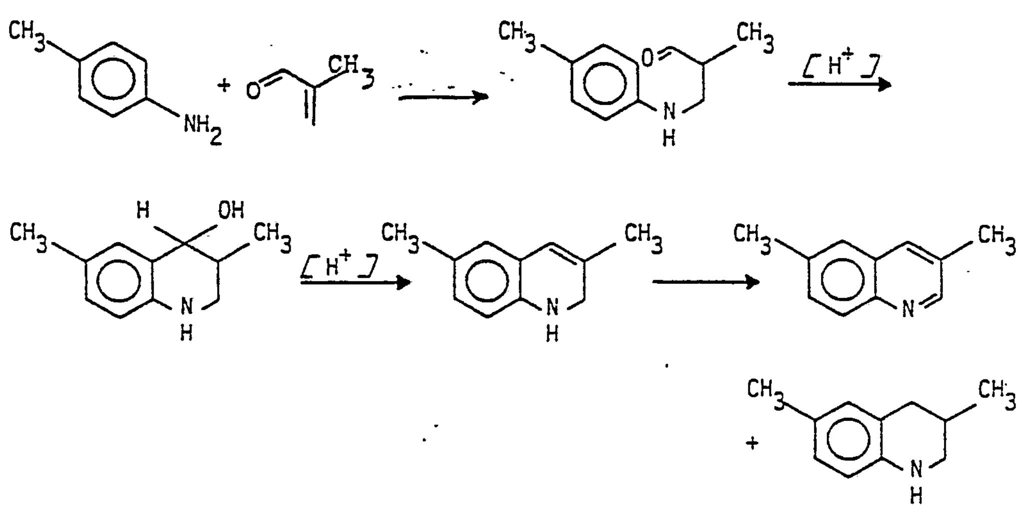


Es ist überraschend, daß in Mineralöl diese mehrstufige Reaktion bei nicht allzu hohen Temperaturen in Anwesenheit von nur katalytischen Mengen Säure mit guten Ausbeuten verläuft. Das Hauptprodukt ist das gewünschte Chinolin oder Chinolinderivat. In Mengen von nur 10 bis 20 %, bezogen auf eingesetztes Anilin erhält man das Tetrahydroderivat, das anschließend in einfacher Weise durch Dehydrierung nach bekannten Methoden in das Zielprodukt überführt werden kann.

Gegenstand der Erfindung ist ein Verfahren zur Herstellung von Chinolinen der Formel

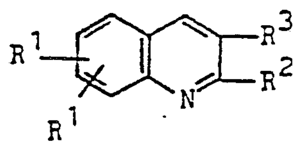

(I)

worin die einzelnen Reste $R^1$, $R^2$ und $R^3$ gleich oder verschieden sein können und jeweils ein Wasserstoffatom oder einen aliphatischen Rest bedeuten, die Reste $R^1$ auch jeweils ein Halogenatom, eine Alkoxygruppe, eine Aminogruppe, eine Monoalkyl- oder eine Dialkylaminogruppe, oder zusammen mit zwei benachbarten Kohlenstoffatomen einen Isoxazolrest, Oxazolrest, Thiazolrest, Isothiazolrest, Furanrest, Thiophenrest, Pyrrolrest, Imidazolrest oder Pyrazolrest bezeichnen, durch Umsetzung von aromatischen Aminen der Formel

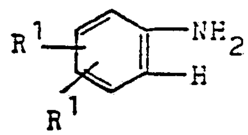

(II)

worin $R^1$ die vorgenannte Bedeutung besitzt, mit Acroleinen oder seinen Acetalen der Formel

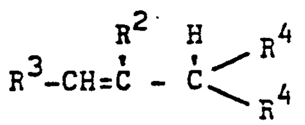

(III)

worin $R^2$ und $R^3$ die vorgenannte Bedeutung besitzen und die einzelnen Reste $R^4$ gleich oder verschieden sein können und jeweils für eine Alkoxygruppe oder zusammen für ein Sauerstoffatom stehen, her. Die Ausgangsstoffe II und III können in stöchiometrischer Menge oder im Überschuß jeder Komponente zur anderen, vorteilhaft in einem Verhältnis von 0,8 bis 4, insbesondere 1 bis 2 Mol Ausgangsstoff III je Mol Ausgangsstoff II, umgesetzt werden. Verwendet man Ausgangsstoffe II, die 2 oder 3 Aminogruppen im Molekül tragen, so wird in der Regel ein Molverhältnis von höchstens 1,5, zweckmäßig 0,8 bis 1,5, vorteilhaft 1 bis 1,5, insbesondere 1,1 bis 1,3 Mol Ausgangsstoff III je Mol Ausgangsstoff II gewählt. Verwendet man ein Verhältnis von mehr als 1,5 Mol Ausgangsstoff III, so bilden sich in mit größeren Mengen an Ausgangsstoff III steigenden Mengen Phenanthroline bzw. entsprechende Triazaverbindungen mit 4 Ringen im Molekül. Bevorzugte Ausgangsstoffe II und III und dementsprechend bevorzugt Endstoffe I sind solche, in deren Formeln die einzelnen Reste $R^1$, $R^2$ und $R^3$ gleich oder verschieden sein können und jeweils ein Wasserstoffatom, eine Carboxylgruppe oder einen Alkylrest mit 1 bis 8, insbesondere 1 bis 4 Kohlenstoffatomen bedeuten, die Reste $R^1$ auch jeweils ein Fluoratom, Chloratom oder Bromatom, eine Aminogruppe, eine Alkoxygruppe, Acylaminogruppe, eine Monoalkyl- oder Dialkylaminogruppe mit jeweils 1 bis 8, insbesondere 1 bis 4 Kohlenstoffatomen je Alkylgruppe, oder zusammen mit zwei benachbarten Kohlenstoffatomen einen Isoxazolrest, Oxazolrest, Thiazolrest, Isothiazolrest, Furanrest, Thiophenrest, Pyrrolrest, Imidazolrest oder Pyrazolrest bezeichnen, die einzelnen Reste $R^4$ gleich oder verschieden sein können und jeweils für eine Alkoxygruppe mit 1 bis 4 Kohlenstoffatomen oder zusammen für ein Sauerstoffatom stehen. Die vorgenannten Reste können noch durch unter den Reaktionsbedingungen inerte Gruppen oder Atome, z.B. Chloratome, Bromatome, Alkylgruppen, Alkoxygruppen mit jeweils 1 bis 4 Kohlenstoffatomen, Nitrogruppen, Acylaminoreste, substituiert sein.

Beispielsweise sind folgende Ausgangsstoffe II geeignet : Anilin, o-Methyl-, o-Ethyl-, o-Propyl-, o-Isopropyl-, o-Butyl-, o-Isobutyl-, o-sek.-Butyl-, o-tert.-Butyl-, o-Methoxy-, o-Ethoxy-, o-Propoxy-, o-Isopropoxy-, o-Butoxy-, o-Isobutoxy-, o-sek.-Butoxy-, o-tert.-Butoxy-, o-Dimethylamino-, o-Diethylamino-, o-Dipropylamino-, o-Dibutylamino-, o-Diisobutylamino-, o-Di-sek.-butylamino-, o-Di-tert.-butylamino-, o-N-Methyl-N-ethylamino-, o-Chlor-, o-Brom-, o-Carboxy-anilin ; anstelle der vorgenannten Dialkylaminogruppen mit entsprechenden Monoalkylaminogruppen oder der unsubstituierten Aminogruppe substituierte Aniline ; durch vorgenannte Gruppen meta-substituierte oder para-substituierte Aniline ; in 2,4-, 2,3-, 2,5-, 3,4- oder 3,5-Stellung durch vorgenannte Gruppen gleich oder verschieden zweifach substituierte Aniline ; in 4-Stellung, 5-Stellung, 6-Stellung oder 7-Stellung durch die Aminogruppe substituiertes Benzofuran, Thionaphthen, Indol, Benzoxazol, Benzpyrazol, Benzimidazol, Benzisoxazol, Benzthiazol, Benzisothiazol.

Folgende Ausgangsstoffe III können beispielsweise verwendet werden : Acrolein ; in 2-Stellung und/oder 3-Stellung durch gleiche oder unterschiedliche Methyl-, Ethyl-, Propyl-, Isopropyl-, Butyl-, Isobutyl-, sek.-Butyl-, tert.-Butyl-gruppen substituierte Acroleine ; entsprechende Dimethyl-, Diethyl-, Dipropyl-, Diisopropyl-, Dibutyl-, Diisobutyl-, Di-sek.-butyl-, Di-tert.-butylacetale vorgenannter Acroleine.

Bevorzugte reaktionsfähige α, β-ungesättigte Aldehyde sind Acrolein, Methacrolein, Crotonaldehyd, Zimtaldehyd und Ethylacrolein.

Die erfindungsgemäße Umsetzung wird vorzugsweise mit sauren Katalysatoren durchgeführt.

Es können sowohl im Mineralöl unlösliche als auch im Mineralöl lösliche Katalysatoren verwendet werden, die demgemäß in Mineralöl gelöst, emulgiert oder suspendiert sind. Die Umsetzung wird im allgemeinen in Gegenwart von anorganischer oder organischer Säure, in der Regel in katalytischen Mengen, vorteilhaft mit einer Menge von 0,001 bis 0,4, insbesondere von 0,005 bis 0,05 Äquivalenten Säure, bezogen auf 1 Mol Ausgangsstoff II, durchgeführt. Anstelle einbasischer Säuren können auch äquivalente Mengen mehrbasischer Säuren zur Anwendung gelangen. Beispielsweise sind folgende Säuren geeignet : Sulfonsäuren wie Benzol- und p-Toluolsulfonsäure, Dodecylbenzolsulfonsäure ; aliphatische Carbonsäuren wie Oxalsäure, Ameisensäure, Essigsäure, Propionsäure, Capronsäure, Caprinsäure, 3,5,5-Trimethylhexansäure, Laurinsäure, Palmitinsäure, Stearinsäure, 2-Ethylhexancarbonsäure, 2-Ethylbuttersäure, 2-Methylbutansäure, Glykolsäure, Milchsäure, Brenztraubensäure, Weinsäure, Caprylsäure, Trimethylessigsäure, Bernsteinsäure, Isovaleriansäure, Valeriansäure, Glutarsäure, Adipinsäure ; cycloaliphatischen, araliphatischen, aromatischen Carbonsäuren wie Benzoesäure, 2,3-, 2,4-, 2,5-, 2,6-Dimethylbenzoesäure, Mellithsäure, Phenylpropionsäure, o-, m-, bzw. p-Chlorbenzoesäure, Cyclohexancarbonsäure, Cyclopentancarbonsäure, Phenylessigsäure, α- bzw. β-Naphthoesäure, Phthalsäure, o-, m-, bzw. p-Totuylsäure, Isophthalsäure, Terephthalsäure, saure Ionenaustauscher oder entsprechende Gemische. Bevorzugt sind Toluolsulfonsäuren, Benzolsulfonsäure, Dodecylbenzolsulfonsäure, Schwefelsäure, Schwefelsäurehalbester wie Alkylschwefelsäure, Phosphorsäure oder deren partiell veresterte Derivate oder Borsäure und deren saure Derivate. Man kann auch Säureanhydride wie Phosphorpentoxid, Schwefeldioxid und Boroxid verwenden. Enthalten die Ausgangsstoffe, insbesondere Ausgangsstoff II, Carboxylgruppen, sind also selbst Säuren, so wird gegebenenfalls keine zusätzliche Säure, sondern der Ausgangsstoff gleichzeitig als Katalysator verwendet ; in solchen Fällen ist ein Molverhältnis von 0,8 bis 4, vorteilhaft 1 bis 2 Mol Ausgangsstoff III je Mol Ausgangsstoff II zweckmäßig.

Die im Mineralöl löslichen Katalysatoren werden dem Mineralöl im allgemeinen in Mengen von 0,01 bis 25 Gew.%, vorzugsweise 0,1 bis 20 Gew.%, insbesondere 1 bis 7 Gew.%, bezogen auf das Mineralöl, zugesetzt.

Die Chinolinsynthese wird im allgemeinen bei Temperaturen von 50 bis 350 °C, vorzugsweise 80 bis 250 °C, insbesondere 100 bis 200 °C durchgeführt.

Als Reaktoren sind z.B. Rührkessel geeignet. Vorteilhaft werden jedoch für das neue Verfahren senkrecht angeordnete zylindrische Reaktoren, wie Glockenbodenkolonnen, Blasensäulen oder Füllkörperkolonnen, verwendet. Der Ausgangsstoff oder die Ausgangsstoffe werden in der Regel gasförmig bzw. flüssig am Boden des mit Mineralöl gefüllten Reaktors zugeführt. Es kann vorteilhaft sein, den verdampften Ausgangsstoff mit einem inerten Gas zu verdünnen. Geeignete inerte Gase sind z.B. Wasserdampf, Kohlendioxid und vorzugsweise Stickstoff.

Die Reaktionsprodukte werden gasförmig am Kopf des Reaktors abgezogen und anschließend zweckmäßig kondensiert. An die Kondensation kann noch eine Reinigungsstufe, z.B. eine Destillation oder Fraktionierung angeschlossen werden.

Das neue Verfahren kann diskontinuierlich oder kontinuierlich durchgeführt werden, wobei jedoch die kontinuierliche Arbeitsweise bevorzugt wird. Bei der kontinuierlichen Arbeitsweise kann es vorteilhaft sein, frisches Mineralöl kontinuierlich zuzuführen und das verbrauchte Lösungsmittel fortlaufend abzuziehen, beispielsweise wenn es sich um eine Reaktion handelt, bei der Crackprodukte und Polymere gebildet werden, so daß die Crackprodukte mit dem Mineralöl ständig aus dem Reaktor ausgeschleust werden.

Eine gleichermaßen bevorzugte Ausführungsform ist die gleichzeitige allmähliche Zuführung der Reaktionskomponenten in das Katalysator-enthaltende Mineralöl, ohne daß die Reaktionsprodukte fortlaufend entfernt werden. Nach Anreicherung der Endprodukte wird der Zulauf der Ausgangsstoffe abgestellt und die Wertprodukte durch Destillation von dem Mineralöl getrennt. Höhersiedende Nebenprodukte verbleiben im Öl. Das Öl kann größtenteils wieder verwendet, ein Teil muß jedoch ausgeschleust werden.

Eine Aufarbeitung und Rückführung des abgezogenen Mineralöls ist in der Regel nicht wirtschaftlich, da das Mineralöl z.B. als Heizöl oder Vakuumgasöl in der Regel wohlfeil zur Verfügung steht. Man wird daher zweckmäßig das abgezogene Crackprodukt enthaltende Mineralöl einer Verfeuerung und dem Reaktor frisches Mineralöl zuführen.

Das neue Verfahren hat gegenüber denen des Standes der Technik wesentliche Vorteile :

Man kommt mit katalytischen Mengen Säuren aus und spart die Neutralisation von großen Mengen Schwefelsäure ein. Polymere und Kondensationsprodukte, die bei Umsetzungen von Aldehyden mit Aminen unvermeidlich sind, verbleiben im Mineralöl, das nicht regeneriert zu werden braucht und das gegebenenfalls nach Abtrennen des Katalysators zweckmäßig der Verfeuerung im Kraftwerk zugeführt wird. Ferner ist das Verfahren sowohl in der technischen Ausführung als auch im Energieverbrauch wesentlich wirtschaftlicher. Schließlich bewirkt die Verbrennung des Reaktionsmediums und der darin enthaltenen Nebenprodukte eine verringerte Umweltbelastung.

Beispiel 1

In der in Figur 1 abgebildeten Apparatur wurden stündlich 123 g Acrolein und 283 g 3-Chlor-2-methylanilin aus den Vorlagen 2a, 2b mittels Dosierpumpen 1a, 1b den Vorverdampfern 3a, 3b zugeführt und dort mit je 30 l/h $N_2$ bei 170 °C verdampft und über eine Zweistoffdüse 4 in den Reaktor 5 eingebracht. Die Reaktionstemperatur betrug 150 °C.

Der Reaktor besteht aus einem Doppelmantelrohr von 1,25 m Länge und einem Durchmesser von 65 mm. Über dem Düseneingang ist in einem Abstand von 3 cm eine Lochplatte mit 5 mm-Bohrungen angeordnet. Der obere Teil des Reaktors ist mit 3 l Glasringen 5 × 5 mm gefüllt.

Im Reaktor befanden sich 1,2 kg Vakuumgasöl. Kp. > 350 °C mit 5 Gew.% Dodecylbenzolsulfonsäure als Katalysator.

Das entstandene Reaktionswasser sowie nicht umgesetztes Acrolein wurde in einem Kühler 6 kondensiert und in einem Auffangbehälter 7 gesammelt. Das entstandene 7-Chlor-8-methylchinolin wurde aus dem Vakuumgasöl des Reaktors durch fraktionierte Destillation gewonnen.

Bezogen auf die pro Stunde zugeführten Ausgangsmaterialien erhielt man 192 g Destillat, $Kp._{2Torr}$ 119 °C, das nach GC 98 Gew.% 7-Chlor-8-methylchinolin enthielt, d.s. 53 %, bezogen auf eingesetztes 3-Chlor-2-methylanilin.

In einer weiteren Fraktion wurden 82,6 g 7-Chlor-8-methyltetrahydrochinolin erhalten, das sich durch Erhitzen mit 2-Chlor-5-nitrotoluol in 7-Chlor-8-methylchinolin überführen läßt. Dadurch erhöhte sich die Ausbeute auf 76 %, bezogen auf eingesetztes 3-Chlor-2-methylanilin.

Beispiel 2

Man verfuhr wie in Beispiel 1 beschrieben, setzte jedoch 1,2 kg Vakuumgasöl Kp. 350 °C mit 1 Gew.% Dodecylbenzolsulfonsäure als Katalysator ein. Bezogen auf die pro Stunde zugeführten Ausgangsmaterialien erhielt man 210 g Destillat $Kp._{3mbar}$ 119 °C mit 98 Gew.% 7-Chlor-8-methylchinolin, entsprechend einer Ausbeute von 58 %, bezogen auf eingesetztes 3-Chlor-2-methylanilin.

In einer weiteren Fraktion wurden 36 g 7-Chlor-8-methyltetrahydrochinolin erhalten, das sich durch Erhitzen mit 2-Chlor-5-nitrotoluol in 7-Chlor-8-methylchinolin oxidieren ließ. Dadurch erhöhte sich die Ausbeute auf 68 %, bezogen auf eingesetztes 3-Chlor-2-methylanilin.

Beispiel 3

Man verfuhr wie in Beispiel 1 beschrieben, setzte jedoch stündlich 187 g Ethylacrolein und 283 g 3-Chlor-2-methylanilin ein. Durch Destillation erhielt man bei $Kp_{3mbar}$ 125 °C 335 g Destillat, das 75 Gew.% 3-Ethyl-7-chlor-8-methylchinolin und 22 Gew.% 3-Ethyl-7-Chlor-8-methyltetrahydrochinolin enthielt. Dies entsprach einer Ausbeute von 60 % des Chinolins, bezogen auf eingesetztes 3-Chlor-2-methylanilin.

Die Tetrahydroverbindung ließ sich durch Oxidation mit 2-Chlor-6-nitrotoluol ebenfalls in 3-Ethyl-7-chlor-8-methylchinolin überführen. Damit stieg die Ausbeute auf 77,5 %.

Beispiel 4

Man verfuhr wie in Beispiel 1 beschrieben, setzte jedoch stündlich 123 g Acrolein und 214 g 3-Methylanilin ein. Durch Destillation erhielt man 140 g Destillat von $Kp_{20mbar}$ 128 °C, das 92 Gew.% 8-Methylchinolin enthielt, entsprechend einer Ausbeute von 45 %, bezogen auf eingesetztes 3-Methylanilin.

Beispiel 5

Man verfuhr wie in Beispiel 1 beschrieben, setzte jedoch stündlich 107 g 4-Toluidin und 84 g Methacrolein ein. Man destillierte bei 5 mbar bis zu einer Sumpftemperatur von 250 °C ab und erhielt durch Kristallisation mit Xylol 110 g 3,6-Dimethylchinolin Fp. 50 °C, entsprechend einer Ausbeute von 70 % der Theorie.

Beispiel 6

Man verfuhr wie in Beispiel 1 beschrieben, setzte jedoch stündlich 130 g 2-Chloranilin und 84 g Crotonaldehyd ein. Nach Destillation aus dem Ölbett bei 5 mbar bis zu einer Sumpftemperatur von 250 °C und Kristallisation des Destillats mit Xylol erhielt man 101 g 8-Chlor-2-methylchinolin, Fp. 64 °C, entsprechend einer Ausbeute von 57 % der Theorie.

**Patentansprüche**

1. Verfahren zur Herstellung von Chinolinen der Formel

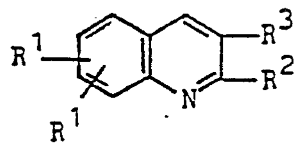

(I)

worin die einzelnen Reste $R^1$, $R^2$ und $R^3$ gleich oder verschieden sein können und jeweils ein Wasserstoffatom oder einen aliphatischen Rest bedeuten, die Reste $R^1$ auch jeweils ein Halogenatom, eine Alkoxygruppe, eine Aminogruppe, eine Monoalkyl- oder eine Dialkylaminogruppe, oder zusammen mit zwei benachbarten Kohlenstoffatomen einen Isoxazolrest, Oxazolrest, Thiazolrest, Isothiazolrest, Furanrest, Thiophenrest, Pyrrolrest, Imidazolrest oder Pyrazolrest bezeichnen, durch Umsetzung von aromatischen Aminen der Formel

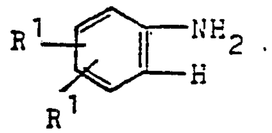

(II)

worin $R^1$ die vorgenannte Bedeutung besitzt, mit Acroleinen oder seinen Acetalen der Formel

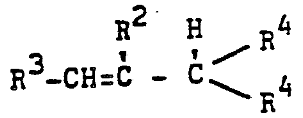

(III)

worin $R^2$ und $R^3$ die vorgenannte Bedeutung besitzen und die einzelnen Reste $R^4$ gleich oder verschieden sein können und jeweils für eine Alkoxygruppe oder zusammen für ein Sauerstoffatom stehen, in Gegenwart von Säure, dadurch gekennzeichnet, daß man das aromatische Amin der Formel II mit dem ungesättigten Aldehyd oder dessen Acetal der Formel III in hochsiedendem Mineralöl bei Temperaturen von 50 bis 350 °C umsetzt, das hochsiedende Mineralöl bei Anreicherung mit Nebenprodukten erneuert und das mit Nebenprodukten angereicherte hochsiedende Mineralöl abzieht.

2. Verfahren nach Anspruch 1, dadurch gekennzeichnet, daß man die Umsetzung in Gegenwart katalytischer Mengen saurer Stoffe durchführt.

3. Verfahren nach Anspruch 1, dadurch gekennzeichnet, daß man als Katalysator eine aromatische Sulfonsäure verwendet.

4. Verfahren nach Anspruch 1, dadurch gekennzeichnet, daß man als Mineralöl hochsiedende Kohlenwasserstoff-Fraktionen verwendet.

5. Verfahren nach Anspruch 1, dadurch gekennzeichnet, daß man als KW-Fraktionen Gasöl, Vakuumgasöl, technisches Weißöl, Schweres Heizöl oder Vakuumrückstand verwendet.

6. Verfahren nach Anspruch 1, dadurch gekennzeichnet, daß man das mit Nebenprodukten angereicherte Mineralöl einer Verfeuerung zuführt.

**Claims**

1. A process for the preparation of a quinoline of the formula

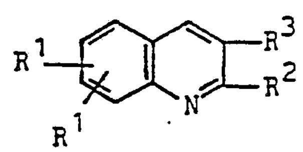

(I)

where the individual radicals $R^1$, $R^2$ and $R^3$ may be identical or different and are each hydrogen or an aliphatic radical, and the radicals $R^1$ may furthermore each be halogen, alkoxy, amino, monoalkylamino or dialkylamino, or, together with two adjacent carbon atoms, may form an isoxazole, oxazole, thiazole, isothiazole, furan, thiophene, pyrrole, imidazole or pyrazole radical, by reacting an aromatic amine of the formula,

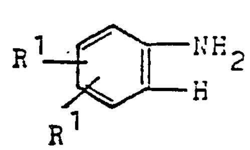

(II)

where $R^1$ has the above meanings, with an acrolein or one of its acetals of the formula

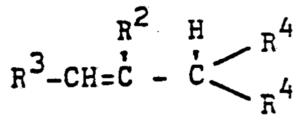

(III)

where $R^2$ and $R^3$ have the above meanings, and the individual radicals $R^4$ may be identical or different and are each alkoxy or together are an oxygen atom, in the presence of an acid, wherein the aromatic amine of the formula II is reacted with the unsaturated aldehyde or its acetal of the formula III in a high-boiling mineral oil at from 50 to 350 °C, the high-boiling mineral oil is replaced when it has become enriched with by-products, and the said mineral oil enriched with by-products is removed.

2. A process as claimed in claim 1, wherein the reaction is carried out in the presence of a catalytic amount of an acidic substance.

3. A process as claimed in claim 1, wherein an aromatic sulfonic acid is used as the catalyst.

4. A process as claimed in claim 1, wherein a high-boiling hydrocarbon fraction is used as the mineral oil.

5. A process as claimed in claim 1, wherein the hydrocarbon fraction used is gas oil, vacuum gas oil, technical white oil, heavy fuel oil or a vacuum residue.

6. A process as claimed in claim 1, wherein the mineral oil enriched with by-products is fed to a combustion unit.

**Revendications**

1. Procédé pour la préparation de quinoléines de formule

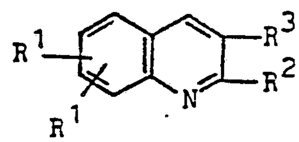

(I)

dans laquelle les groupes individuels $R^1$, $R^2$ et $R^3$ peuvent être identiques ou différents, et représentent chacun un atome d'hydrogène ou un groupe aliphatique, les groupes $R^1$ représentant également chacun un atome d'halogène, un groupe alcoxy, un groupe amino, un groupe monoalkyl- ou dialkylamino, ou en commun avec deux atomes de carbone voisins, un groupe isoxazol, un groupe oxazol, un groupe thiazol, un groupe isothiazol, un groupe furane, un groupe thiophène, un groupe pyrrol, un groupe imidazol ou pyrazol, en faisant réagir des amines aromatiques de formule

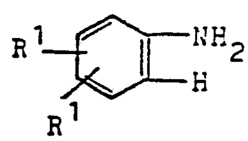

(II)

dans laquelle $R^1$ a la signification mentionnée plus haut, avec l'acroléine ou ses acétals de formule

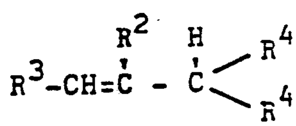

(III)

dans laquelle $R^2$ et $R^3$ ont la signification mentionnée plus haut, et les groupes individuels $R^4$ peuvent être identiques ou différents, et représentent chacun un groupe alcoxy ou ensemble un atome d'oxygène, en présence d'acide, caractérisé en ce qu'on fait réagir l'amine aromatique de formule II avec l'aldéhyde insaturé ou son acétal de formule III, dans une huile minérale à point d'ébullition élevé, à une température de 50 à 350 °C, qu'on renouvelle l'huile minérale à point d'ébullition élevé par enrichissement avec un sous-produit, et qu'on soutire l'huile minérale à point d'ébullition élevé enrichie en sous-produit.

2. Procédé selon la revendication 1, caractérisé en ce que la réaction est effectuée en présence de quantités catalytiques de matières acides.

3. Procédé selon la revendication 1, caractérisé en ce que comme catalyseur on utilise un acide sulfonique aromatique.

4. Procédé selon la revendication 1, caractérisé en ce que comme huile minérale on utilise des fractions d'hydrocarbure à point d'ébullition élevé.

5. Procédé selon la revendication 1, caractérisé en ce que comme fraction d'hydrocarbure on utilise du gasoil, du gasoil obtenu par distillation sous vide, du white-spirit technique, du fuel-oil lourd ou des résidus de distillation sous vide.

6. Procédé selon la revendication 1, caractérisé en ce que l'huile minérale enrichie en sous-produit est amenée pour être utilisée dans une combustion.

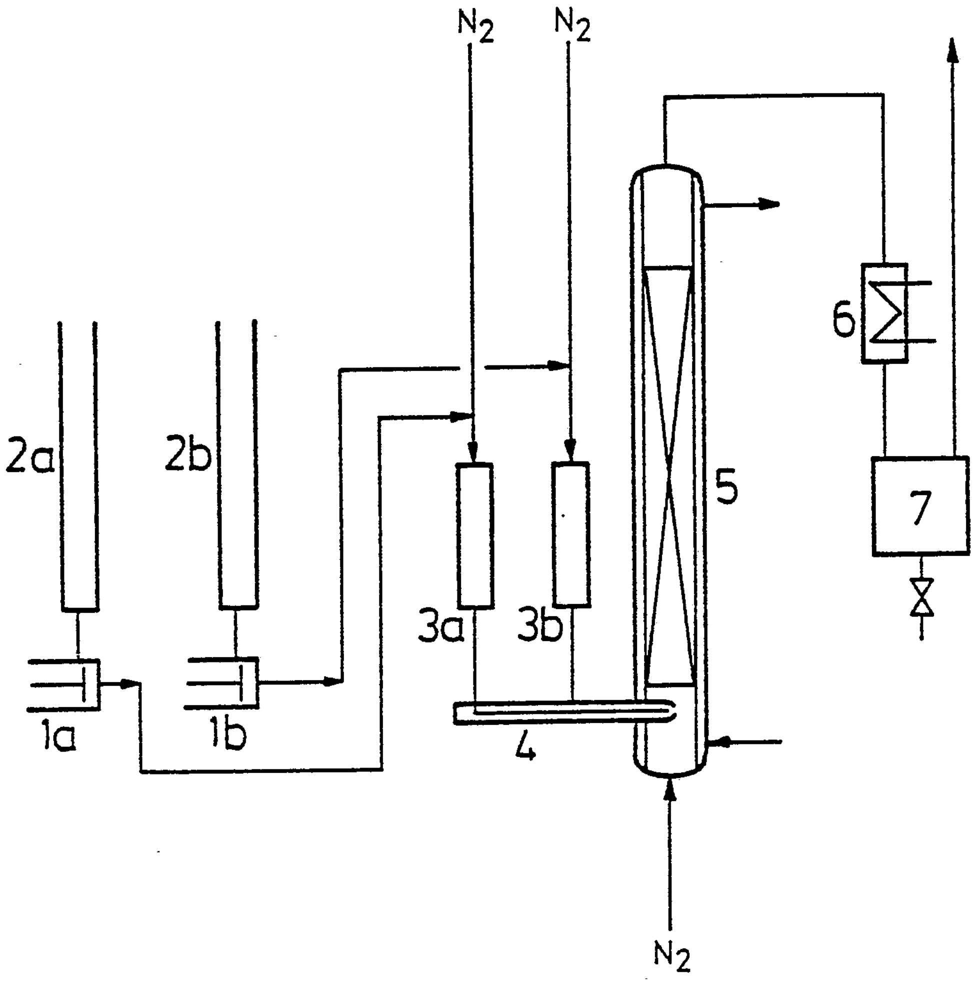


FIG. 1